# EUROPÄISCHE PATENTANMELDUNG

(11) **EP 4 545 646 A1**
(43) Veröffentlichungstag der Anmeldung: **30.04.2025**
(21) Anmeldenummer: 23206404.8
(22) Anmeldetag: 27.10.2023
(51) Int. Cl.: C12P 19/02, C12P 19/24, A23L 27/30, C12N 9/04, C12N 9/02, C12N 9/90

(54) **VERFAHREN ZUR HERSTELLUNG VON D-TALITOL**

(71) Anmelder: Annikki GmbH, 8074 Raaba-Grambach (AT)
(72) Erfinder: Dupont, Maria, 8074 Raaba-Grambach (AT); Staunig, Nicole, 8074 Raaba-Grambach (AT)
(74) Vertreter: Schwarz & Partner Patentanwälte GmbH

(57) **Zusammenfassung**

Verfahren zur Herstellung einer wässerigen Lösung enthaltend D-Talitol, indem aus D-Fructose, welche in einer wässerigen Lösung gelöst vorliegt, durch Behandeln mit einer Epimerase *in vitro* D-Psicose gebildet wird, wonach die D-Psicose durch Behandeln mit einer NAD(P)H-abhängigen Oxidoreduktase *in vitro* unter Bildung von NAD(P)' zu D-Talitol reduziert wird und das NAD(P)⁺ mittels Formiat und einer Formiat-Dehydrogenase unter Bildung von CO₂ zu NAD(P)H reduziert wird, wonach die Epimerase, die Oxidoreduktase und die Formiat-Dehydrogenase abgetrennt werden.

## Beschreibung

Die vorliegende Erfindung betrifft die Herstellung von _{D}-Talitol und einer wässerigen Lösung von _{D}-Talitol.

### Hintergrund der Erfindung

Der sechswertige Zuckeralkohol _{D}-Talitol (auch bekannt als _{D}-Altritol) gehört aufgrund seines geringen natürlichen Vorkommens (z.B. in Braunalgen wie *Himanthalia elongata* (Reed et al., 1995)) zu den sogenannten seltenen Zuckern. Aufgrund des Fehlens einer einfachen Herstellungsmethode für den Zuckeralkohol sind die Anwendungsmöglichkeiten von _{D}-Talitol nicht gut untersucht (Li et al., 2013).

Fest steht jedoch, dass _{D}-Talitol in der sogenannten Izumoring-Strategie eine wichtige Rolle einnimmt, da dessen Oxidation zu den Ketohexosen _{D}-Tagatose und _{D}-Psicose sowie zu den Aldohexosen _{D}-Altrose und _{D}-Talose führt, die ebenfalls zu den seltenen Zuckern gezählt werden (Izumori, 2006). Die beiden Ketohexosen _{D}-Tagatose und _{D}-Psicose zeichnen sich durch ihren süßen Geschmack bei einem deutlichen geringeren Energiegehalt (bezogen auf Saccharose) aus (Jiang et al., 2020; Oh, 2007; Roy et al., 2018), was beide Zucker als Süßungsmittel für die Lebensmittelindustrie besonders interessant macht.

Chemisch kann _{D}-Talitol durch Hydrierung von _{D}-Psicose mit Raney-Nickel oder Metallen der Platingruppe als Katalysator gewonnen werden, jedoch entsteht als Nebenprodukt Allitol (Epimer von _{D}-Talitol), welches chromatographisch abgetrennt werden muss (US 2004/0143024 A1).

Eine elegantere Methode ist die biotechnologische Herstellung von _{D}-Talitol. In der Izumoring-Strategie ist _{D}-Talitol in zwei enzymatischen Schritten ausgehend von der häufig vorkommenden _{D}-Fructose erreichbar (Izumori, 2006).

Im ersten Schritt wird _{D}-Fructose mit einer Ketose-3-Epimerase zu _{D}-Psicose epimerisiert (Izumori et al., 1993). Ketose-3-Epimerasen lassen sich je nach Substratspezifität in drei Gruppen einteilen: 1) _{D}-Tagatose-3-Epimerase (DTE), 2) _{D}-Psicose-3-Epimerase (DPE) oder _{D}-Allulose-3-Epimerase (DAE) und 3) _{L}-Ribulose-3-Epimerase (LRE) (Zhang et al., 2016; Jiang et al., 2020).

Die Umsetzung von _{D}-Fructose zu _{D}-Psicose mittels Ketose-3-Epimerasen läuft jedoch nicht vollständig ab, vielmehr bildet sich ein Gleichgewichtsverhältnis zwischen beiden Epimeren aus. Je nach Reaktionsbedingungen (Temperatur zwischen 40 und 70 °C, pH-Wert zwischen 6 und 11) liegt dieses zwischen 80:20 und 62,5:37,5 (_{D}-Fructose : _{D}-Psicose). Viele der Epimerasen benötigen außerdem ein zweiwertiges Metallion wie Mn2⁺ oder Co²⁺ (toxisch) als Kofaktor (Zhang et al., 2016; Jiang et al., 2020).

Der zweite Schritt beinhaltet eine stereoselektive Reduktion der Ketogruppe von _{D}-Psicose zu _{D}-Talitol, welche beispielsweise mit dem halotoleranten Hefe-Stamm *Candida famata* R28 (Sasahara et al., 1998) oder mit weiteren Pilzen (JP 2020039301 A; JP 2006166718 A) bewerkstelligt werden kann.

Für die Herstellung von Allitol (Epimer von _{D}-Talitol) sind enzymatische Verfahren ausgehend von _{D}-Fructose beschrieben. Takeshita et al. (2000) verwendeten ein Enzymsystem bestehend aus einer Epimerase (DTE) mit einer Ribitol-Dehydrogenase (RDH) und Formiat-Dehydrogenase (FDH; oxidiert Formiat zu CO₂) als Regenerationsenzym für den Kofaktor Nicotinamidadenindinukleotid (NADH), um _{D}-Fructose (10 g/l) in 48 h zu Allitol (100% Umsatz) umzuwandeln. Wang et al. (2023) beschreiben ein ähnliches System, welches einen E. coli-Ganzzell-Biokatalysator mit einer DPE, RDH sowie einer FDH beinhaltet. Auf diese Weise wurde _{D}-Fructose (500 mM = 90 g/l) innerhalb von 12 h bei 37 °C und pH 6 unter Einsatz von 1000 mM Natrium-Formiat und 0,5 mM NAD⁺ zu 452 mM Allitol umgewandelt (Umsatz 90,4%).

Zum jetzigen Zeitpunkt sind noch keine enzymatischen Verfahren für die Umsetzung von _{D}-Psicose zu _{D}-Talitol beschrieben.

Hier setzt nun die Aufgabe der vorliegenden Erfindung an. Die Erfindung möchte basierend auf den zuvor beschriebenen biotechnologischen Routen ein effizientes enzymatisches Verfahren zur Herstellung von _{D}-Talitol zur Verfügung stellen.

### Detaillierte Beschreibung der Erfindung

Die Aufgabe zur Herstellung von _{D}-Talitol wird erfindungsgemäß gelöst, indem aus _{D}-Fructose, welche in einer wässerigen Lösung gelöst vorliegt, durch Behandeln mit einer Epimerase *in vitro* _{D}-Psicose gebildet wird, wonach die _{D}-Psicose durch Behandeln mit einer NAD(P)H-abhängigen Oxidoreduktase *in vitro* unter Bildung von NAD(P)⁺ zu _{D}-Talitol reduziert wird und das NAD(P)⁺ mittels Formiat und einer Formiat-Dehydrogenase unter Bildung von CO₂ zu NAD(P)H reduziert wird, wonach die Epimerase, die Oxidoreduktase und die Formiat-Dehydrogenase abgetrennt werden.

Aus der erhaltenen Lösung kann _{D}-Talitol durch Kristallisation gewonnen werden.

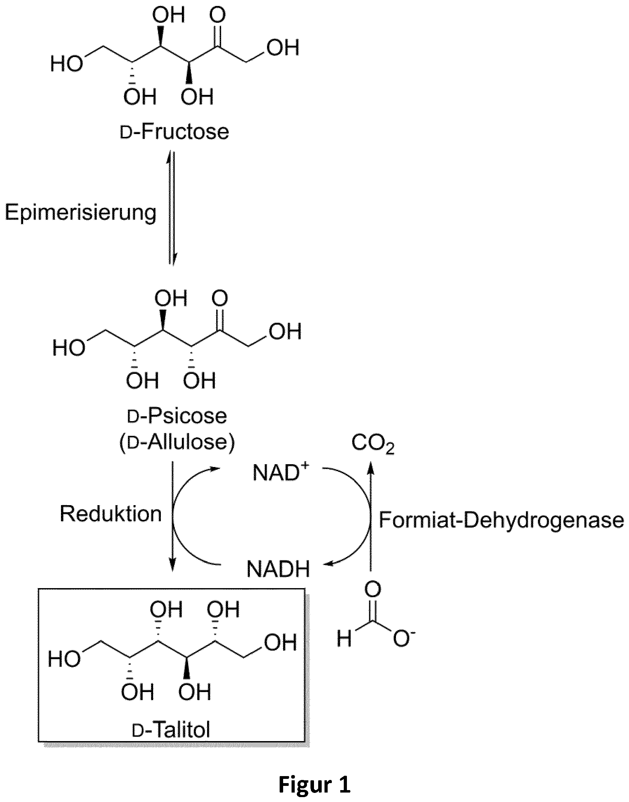

In der beiliegenden Figur 1 ist das Reaktionsschema des erfindungsgemäßen Verfahrens wiedergegeben.

Eine weitere bevorzugte Variante der erfindungsgemäßen Verfahren ist dadurch gekennzeichnet, dass sie als Eintopf-Reaktion ohne Isolierung von Zwischenprodukten durchgeführt werden.

Die Abtrennung der Enzyme kann z.B. durch Zentrifugieren oder Ultrafiltration bewerkstelligt werden.

Die besonders bevorzugte Konzentration von _{D}-Fructose beträgt 50 - 250 g/l.

Der besonders bevorzugte Temperaturbereich für den ersten Schritt (Epimerisierung und Reduktion) liegt zwischen 25 und 40 °C.

Der besonders bevorzugte pH-Bereich für die Reaktion liegt zwischen 6,0 und 8,5.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind. Zudem können die Enzyme auch in lyophilisierter oder sprühgetrockneter oder immobilisierter Form im erfindungsgemäßen Verfahren eingesetzt werden. Die erfindungsgemäß eingesetzten Enzyme können auch in einer im Wesentlichen gereinigten Form verwendet werden, so dass die Enzympräparate keine oder im Wesentlichen keine Bestandteile umfassen, die von den Zellen bzw. der Kultur, in der diese exprimiert wurden, stammen. Die beispielsweise durch rekombinante Expression (z.B. in E. coli) hergestellten Enzyme können durch Verfahren, die im Stand der Technik bekannt sind, gereinigt werden, um nach u.a. Ultrafiltration und DNA-Entfernung auch in und zur Herstellung von Lebensmitteln eingesetzt zu werden.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme* & *Herstellung der Lysate* für Details).

In einer weiteren Variante können die Enzyme auch mit einem wasserlöslichen Polymer wie Polyethylenglycol am N-Terminus modifiziert, in oder auf einer festen Matrix immobilisiert oder Teil eines Fusionsproteins sein.

In einer besonders bevorzugten Ausführung des Verfahrens werden für die Konversion des Startmaterials nur Enzyme aus den Enzymgruppen Epimerasen und Oxidoreduktasen verwendet, wobei eines oder mehrere dieser Enzyme aus jeder dieser Gruppen ausgewählt werden.

Die im Verfahren verwendete Epimerase kann aus einer der Gruppen EC 5.1.3.30 (_{D}-Psicose-3-Epimerase) oder EC 5.1.3.31 (_{D}-Tagatose-3-Epimerase/_{L}-Ribulose-3-Epimerase) stammen, wobei die erstgenannte besonders bevorzugt ist.

Die NAD(P)H-abhängige Oxidoreduktase zur Reduktion von _{D}-Psicose zu _{D}-Talitol umfasst vorzugsweise eine Aminosäuresequenz, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

SEQ ID Nr. 1: 
ATGAACGATCCCGTTCTGGAAGCCAGACCGATTTTGGAGAGATTTCGCCTCGACGGCCGCGTGGCGC
TGGTGACCGGCGGTGGGCAGGGCATCGGCCGCGCCTTCGCCCATGCGCTGTGCGAGGCAGGCGCCG
CAGTGGCCGTCGTCGATCTGCGCCTGGACCTGGCCGAGGAAGTGGCGCATGAGCTGGTAAACAAGC
AGATCGACGCCATCGCAATTCAGGCCGATGTGACGAAGCCCGATCAGGTGCAGACGATGGTTGATGC
CATTCTGTCCAGGTGGGGGACGTTGACGATCGGTGTCAACAACGCCGGCATCGGCTTGTGGGCGGAT
GCCGAAAGCATGGCATACACAGACTGGCTGCGCGTGATCGATATTGATCTGAACTCGGTTTTCCTCTG
CGCCCAGGCTGAAGCGAGGGTCATGCTGCCGGCCGGCTACGGCAAGATCATCAACACGGCGTCGAT
GTCCGGCCATATCAGCAACACGCCGCAAAATCAGGCTGCTTACAACACGGCCAAGGCTGGCGTGATC
CATCTGACGCGCAGCCTTGCCGCGGAATGGGCCAAGCGGGGAGTGCGCGTCAACAGCATCAGCCCC
GGCTATACGCGCACCAAATTGGTTGACGACCTGCTCGCCACGCCGATCGGACAGACGATGTTGCCCA
CCTGGATGGGCATGACGCCCATGGGACGAATGGCCGAGGTCACCGATCTGCAAGGCGCCGTCGTCT
ACCTGGCGTCGTCGGCGTCCGACTATATGACCGGCCATGACATGGTGATTGACGGCGGCTATTGTTG
CTGGTAA
SEQ ID Nr. 2:

MNDPVLEARPILERFRLDGRVALVTGGGQGIGRAFAHALCEAGAAVAVVDLRLDLAEEVAHELVNKQIDA
IAIQADVTKPDQVQTMVDAILSRWGTLTIGVNNAGIGLWADAESMAYTDWLRVIDIDLNSVFLCAQAEA
RVMLPAGYGKIINTASMSGHISNTPQNQAAYNTAKAGVIHLTRSLAAEWAKRGVRVNSISPGYTRTKLVD
DLLATPIGQTMLPTWMGMTPMGRMAEVTDLQGAVVYLASSASDYMTGHDMVIDGGYCCW

Die Oxidoreduktase zur Bildung von _{D}-Talitol aus _{D}-Psicose umfasst vorzugsweise eine Aminosäuresequenz, die eine Identität zu SEQ ID Nr. 2 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Oxidoreduktase zur Bildung von _{D}-Talitol aus _{D}-Psicose die Aminosäuresequenz SEQ ID Nr. 2 oder besteht aus dieser.

Alternativ, umfasst die Oxidoreduktase zur Bildung von _{D}-Talitol aus _{D}-Psicose vorzugsweise eine Aminosäuresequenz, die durch eine Nukleinsäure kodiert wird, die eine Identität zu SEQ ID Nr. 1 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Oxidoreduktase zur Bildung von _{D}-Talitol aus _{D}-Psicose kodiert, die Nukleinsäuresequenz SEQ ID Nr. 1 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990) verwendet. Die BLAST-Software-Reihe enthält verschiedene Programme, einschließlich eines als "BLAST 2 Sequences" genannten Werkzeuges, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 11, eine Erwartung (E) von 10, M = 5, N = -4 und einen Vergleich beider Stränge. Für Aminosäuresequenzen verwendet das blastp-Programm als Vorgaben eine Wortlänge von 3 und eine Erwartung (E) von 10 und die BLOSUM62-Scoring-Matrix (Henikoff & Henikoff, 1989), Alignments (B) von 50, Erwartung (E) von 10, M = 5, N = -4.

Alternativ, umfasst die Oxidoreduktase zur Bildung von _{D}-Talitol aus _{D}-Psicose vorzugsweise eine Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet. Wie hierin verwendet, beziehen sich die stringenten Bedingungen auf Bedingungen, unter denen sogenannte spezifische Hybride, jedoch keine unspezifischen Hybride gebildet werden. Beispielsweise umfassen die stringenten Bedingungen eine Hybridisierung in 6xSSC (Natriumchlorid/Natriumcitrat) bei 45°C und dann Waschen mit 0,2 bis 1xSSC, 0,1% SDS bei 50 bis 65°C; oder derartige Bedingungen können eine Hybridisierung in 1xSSC bei 65 bis 70°C und dann Waschen mit 0,3xSSC bei 65 bis 70°C umfassen. Die Hybridisierung kann durch herkömmlich bekannte Verfahren, wie etwa jene, die von J. Sambrook et al. in Molecular Cloning, A Laboratory Manual, 2nd Ed., Cold Spring Harbor Laboratory (1989), beschrieben sind, durchgeführt werden.

Ein weiterer Aspekt der vorliegenden Erfindung betrifft ein Verfahren zur Herstellung von _{D}-Talitol oder _{D}-Psicose umfassend den Schritt des Behandelns von _{D}-Psicose mit einer Oxidoreduktase, die eine Aminosäuresequenz umfasst, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

Ein noch weiterer Aspekt der vorliegenden Erfindung betrifft eine Oxidoreduktase zur Reduktion von _{D}-Psicose zu _{D}-Talitol umfassend eine Aminosäuresequenz, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

Das bei der Reduktion von _{D}-Psicose zu _{D}-Talitol entstehende NAD(P)⁺ wird mittels Formiat und einer Formiat-Dehydrogenase unter Bildung von CO₂ zu NAD(P)H reduziert (Kofaktor-Regeneration).

Besonders bevorzugt wird eine Formiat-Dehydrogenase eingesetzt, welche die Aminosäuresequenz SEQ ID Nr. 4 umfasst oder daraus besteht, oder ein funktionelles Fragment dieser Formiat-Dehydrogenase. Die bevorzugt eingesetzte Formiat-Dehydrogenase wird vorzugsweise durch die Nukleinsäuresequenz SEQ ID Nr. 3 kodiert. Ein "funktionelles Fragment" der Formiat-Dehydrogenase umfasst eine N-terminal und/oder C-terminal trunkierte Variante der Formiat-Dehydrogenase mit der Aminosäuresequenz SEQ ID Nr. 4, die zumindest 50%, vorzugsweise zumindest 60%, noch mehr bevorzugt zumindest 70%, noch mehr bevorzugt zumindest 80%, noch mehr bevorzugt zumindest 90%, noch mehr bevorzugt zumindest 95%, Enzymaktivität im Vergleich zur nicht trunkierten Formiat-Dehydrogenase aufweist.
SEQ ID Nr. 3: 
ATGGCGAAAATACTTTGCGTTCTCTATGACGATCCGGTCGACGGCTACCCGAAGACCTATGCGCGCG
ACGACCTGCCGAAGATCGACCACTATCCGGGCGGGCAGACGCTGCCCACGCCCAAGGCGATCGACTT
CACGCCGGGCGCGCTGCTCGGCTCGGTCTCCGGCGAGCTCGGCCTGCGCAAATACCTGGAAGCCAAC
GGCCATACCTTCGTCGTCACCTCCGATAAGGACGGCCCGGATTCGGTGTTCGAGAGGGAACTCGTCG
ACGCCGACGTGGTGATCTCGCAGCCCTTCTGGCCGGCCTATCTGACGCCCGAGCGCATCGCCAAGGC
GAAGAACCTGAAGCTCGCGCTCACCGCCGGCATCGGCTCCGATCATGTCGATCTTCAGTCAGCTATCG
ACCGTGGCATCACTGTGGCCGAAGTCACATATTGCAACTCGATCAGCGTCGCCGAGCACGTGGTGAT
GATGATCCTCGGCCTGGTACGAAACTACATTCCCTCGCATGACTGGGCGCGCAAGGGCGGCTGGAAC
ATAGCCGACTGCGTAGAGCACTCCTACGACCTCGAGGGCATGACCGTCGGCTCGGTGGCCGCCGGCC
GCATCGGCCTCGCCGTGCTGCGCCGCCTCGCGCCGTTCGACGTGAAGCTGCACTATACCGACCGCCA
CCGTCTGCCAGAAGCGGTCGAGAAGGAGCTGGGCCTCGTCTGGCACGATACCCGCGAGGACATGTA
CCCGCATTGCGACGTGGTCACGCTCAACGTGCCGCTGCACCCCGAAACCGAGCACATGATCAATGAC
GAGACGCTGAAGCTGTTCAAGCGCGGCGCCTATATCGTCAACACCGCCCGCGGCAAGCTCGCCGACC
GCGACGCCATCGTCCGCGCGATCGAGAGCGGGCAGCTCGCGGGCTATGCCGGCGACGTGTGGTTCC
CGCAGCCGGCTCCGAAGGACCACCCCTGGCGCACCATGAAGTGGGAAGGCATGACGCCGCACATCT
CCGGCACCTCGCTCTCTGCCCAGGCGCGCTACGCGGCGGGCACGCGCGAGATCCTCGAATGCTTCTT
CGAGGGCCGGCCGATCCGCGACGAGTACCTGATCGTGCAGGGCGGCGCGCTCGCCGGCACCGGCGC
GCATTCCTACTCGAAGGGCAATGCGACCGGCGGTTCGGAAGAGGCCGCGAAGTTCAAGAAGGCTGG
CTGA
SEQ ID Nr. 4:

MAKILCVLYDDPVDGYPKTYARDDLPKIDHYPGGQTLPTPKAIDFTPGALLGSVSGELGLRKYLEANGHTFV
VTSDKDGPDSVFERELVDADVVISQPFWPAYLTPERIAKAKNLKLALTAGIGSDHVDLQSAIDRGITVAEVT
YCNSISVAEHVVMMILGLVRNYIPSHDWARKGGWNIADCVEHSYDLEGMTVGSVAAGRIGLAVLRRLAP

FDVKLHYTDRHRLPEAVEKELGLVWHDTREDMYPHCDVVTLNVPLHPETEHMINDETLKLFKRGAYIVNT
ARGKLADRDAIVRAIESGQLAGYAGDVWFPQPAPKDHPWRTMKWEGMTPHISGTSLSAQARYAAGTR
EILECFFEGRPIRDEYLIVQGGALAGTGAHSYSKGNATGGSEEAAKFKKAG

Gemäß einer weiteren bevorzugten Ausführungsform der vorliegenden Erfindung umfasst die zur Kofaktor-Regeneration eingesetzte Formiat-Dehydrogenase eine Aminosäuresequenz, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

Die Formiat-Dehydrogenase umfasst vorzugsweise eine Aminosäuresequenz, die eine Identität zu SEQ ID Nr. 4 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist.

Alternativ, umfasst die Formiat-Dehydrogenase vorzugsweise eine Aminosäuresequenz, die durch eine Nukleinsäure kodiert wird, die eine Identität zu SEQ ID Nr. 1 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist.

Ein weiterer Aspekt der vorliegenden Erfindung betrifft eine wässrige Lösung enthaltend _{D}-Talitol oder _{D}-Talitol bzw. eine Zusammensetzung umfassend _{D}-Talitol erhältlich nach einem erfindungsgemäßen Verfahren.

### Materialien

_{D}-Talitol wurde von TCI, _{D}-Psicose wurde von TCI und Hunan Garden Naturals Inc. (China), _{D}-Fructose, NADPH-Tetranatriumsalz, und Methanol wurden von PanReac AppliChem (ITW Reagents), Zinkchlorid, sowie IPTG (Isopropyl-β-_{D}-thiogalactopyranosid) wurden von Sigma-Aldrich, Magnesiumchlorid-Hexahydrat, Kaliumdihydrogenphosphat, di-Kaliumhydrogenphosphat, NAD⁺, NADH-Dinatriumsalz, NADP⁺-Dinatriumsalz sowie Natriumdodecylsulfat (SDS) wurden von Carl Roth und Triethanolamin (TEA) wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia* coli-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente E. coli-Zellen Top10F transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTGinduzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels GEA-Aufschlusses

Zur Herstellung einer Zell-Suspension (Volumen > 50 ml) wurde das nach obigen Verfahren hergestellte Zellpellet in einem geeigneten Gefäß eingewogen und in Puffer (siehe Tabelle 1 für verwendete Puffer-Systeme) gelöst. Der Massenanteil an Biomasse beträgt üblicherweise 20%, der Rest entfällt auf den Puffer.

Zum Zell-Aufschluss wurde ein Lab Homogenizer PandaPLUS 2000 von GEA verwendet (Druck ca. 1000 bar).

Die Homogenate wurden 10 min lang bei 4 °C und 16000 rpm zentrifugiert (Hermle Z446 K Zentrifuge), um die unlöslichen Zellfragmente abzutrennen und das Lysat zu erhalten.

**Tabelle 1. Enzymklassen, Spenderorganismen und Aufschlussbedingungen für die in den Beispielen verwendeten Enzyme.**

| **Enzymtyp (EC-Klasse)** | **katalysierte Reaktion** | **Aufschlussbedingungen** | **Literatur/SEQ ID Nr.** |
|---|---|---|---|
| D-Psicose-3-Epimerase (EC 5.1.3.30) | D-Fructose → D-Psicose | GEA (20% Biomasse in 100 mM TEA-HCI pH 7 + 2 mM MgCl₂) | (Mu et al., 2011; Chan et al., 2012) |
| Oxidoreduktase I | D-Psicose → D-Talitol | GEA (20% Biomasse in 100 mM TEA-HCI pH 7) | SEQ ID Nr. 2 |
| Formiat-Dehydrogenase (EC 1.17.1.9) | Formiat + NAD⁺ → CO₂ + NADH | GEA (20% Biomasse in 100 mM TEA-HCI pH 7) | SEQ ID Nr. 4 |

### Analytische Methoden

### High Performance Liquid Chromatography

Zur Quantifizierung von _{D}-Talitol, _{D}-Psicose und _{D}-Fructose mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RPM-Monosaccharide Pb+2 (8%) Säule mit entsprechender Vorsäule verwendet und mit Reinstwasser isokratisch eluiert.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bio-one Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH bzw. NADPH bei 340 nm (ε = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit dem nachfolgenden Beispiel werden bevorzugte Varianten des erfindungsgemäßen Verfahrens noch näher beschrieben. Die in diesem Beispiel eingesetzten Lysate wurden nach den oben beschriebenen Verfahren hergestellt.

### Beispiel

### Herstellung von _{D}-Talitol aus _{D}-Fructose

Die Reaktion wurde in einem Labfors 5 Tisch-Bioreaktor (Infors AG) durchgeführt. Als Gefäß wurde ein Glasreaktor (Volumen 3,4 I) mit aufgesetztem Rührwerk, pH-Elektrode und O₂-Sensor verwendet. Die pH-Kontrolle erfolgte durch die Zudosierung von 1M NaOH oder 1M H₂SO₄.

Zu Beginn wurden 17,5 g _{D}-Fructose, 125 ml deionisiertes Wasser sowie 4,2 ml eines 500 mM TEA-HCI-Puffers (pH 7,5) im Reaktor vorgelegt und unter Rühren auf 35 °C gebracht. Um Schaumbildung im Reaktor zu unterbinden bzw. zu reduzieren wurde ein Antischaummittel zugesetzt (Glanapon 2000; Bussetti und Co., GmbH, Österreich).

Zum Start der Reaktion wurden 17,5 ml _{D}-Psicose-3-Epimerase-Lysat zugesetzt. Danach wurden 4,7 kU Oxidoreduktase I-Lysat, 5,5 kU Formiat-Dehydrogenase-Lysat, 3,5 ml einer 10 mM NAD⁺-Lösung sowie 45,5 ml einer 5 M Natrium-Formiat-Lösung eingebracht. Zur Entfernung des durch die Formiat-Dehydrogenase gebildeten CO₂ wurde Luft in die Reaktionsmischung eingeblasen.

Während des Betriebs wurden laufend Proben von der Reaktorlösung genommen, die folgendermaßen analysiert wurden: 100 µl der Reaktorlösung wurden mit 200 µl Methanol versetzt und im Eppendorf Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (Rl-Detektion) vermessen.

Nach 26 h wurden 99% der _{D}-Fructose (50 g/l) zu _{D}-Talitol umgesetzt (gefundene Konzentration: 34,4 g/l).

Im Anschluss wurde der Reaktorinhalt unter Rührung für 30 min auf 70 °C erhitzt. Die heiße Mischung wurde durch einen Faltenfilter (Schleicher & Schuell 595 ½, 185 mm) filtriert. Das Filtrat wurde mit Aktivkohle für 30 min bei 50 °C gerührt, wonach die Aktivkohle abfiltriert wurde (Faltenfilter Schleicher & Schuell 595 ½, 185 mm). Die farblose Lösung wurde mit einem Mischbett-Ionentauscherharz (Amberlite MB20) für 30 min bei 50 °C behandelt, welches danach durch Filtration (Faltenfilter Schleicher & Schuell 595 ½, 185 mm) entfernt wurde. Am Rotationsverdampfer (60 °C, 150 - 60 mbar) wurde die Lösung bis zu einer Konzentration von ca. 600 g/l _{D}-Talitol aufkonzentriert. Der entstandene Sirup wurde über Nacht bei 4 °C im Kühlschrank inkubiert. Da keine Kristallbildung beobachtet werden konnte, wurde ein Impfkristall zugesetzt und der Sirup wieder über Nacht bei 4 °C im Kühlschrank gelagert. Der Sirup wurde mit demselben Volumen an IPA versetzt und bei -20 °C im Tiefkühlschrank gelagert, um die Kristallisation auszulösen. Die farblosen Kristalle wurden nach Anlegen eines Vakuums durch eine Glasfritte (P4, 10-16 µm) abfiltriert und 24 h bei 50 °C im Vakuumtrockenschrank getrocknet. Eine HPLC-Analyse der Kristalle (50 % Ausbeute) ergab, dass es sich bei dem Produkt um _{D}-Talitol (Reinheit 94%) handelte.

### Literatur

Reed, R. H., Wright, P. J., Chudek, J. A., & Hunter, G. (1995). Turnover of hexitols in the marine macroalga Himanthalia elongata (Phaeophyta, Fucales). European Journal of Phycology, 30(3), 169-177. https://doi.org/10.1080/09670269500650951
Li, Z., Gao, Y., Nakanishi, H., Gao, X., & Cai, L. (2013). Biosynthesis of rare hexoses using microorganisms and related enzymes. Beilstein Journal of Organic Chemistry, 9, 2434-2445. https://doi.org/10.3762/bjoc.9.281
Izumori, K. (2006). Izumoring: a strategy for bioproduction of all hexoses. Journal of Biotechnology, 124(4), 717-722. https://doi.org/10.1016/j.jbiotec.2006.04.016
Jiang, S., Xiao, W., Zhu, X., Yang, P., Zheng, Z., Lu, S., Jiang, S., Zhang, G., & Liu, J. (2020). Review on D-Allulose: In vivo Metabolism, Catalytic Mechanism, Engineering Strain Construction, Bio-Production Technology. Frontiers in Bioengineering and Biotechnology, 8, 26. https://doi.org/10.3389/fbioe.2020.00026
Oh, D.-K. (2007). Tagatose: properties, applications, and biotechnological processes. Applied Microbiology and Biotechnology, 76, 1-8. https://doi.org/10.1007/s00253-007-0981-1
Roy, S., Chikkerur, J., Roy, S. C., Dhali, A., Kolte, A. P., Sridhar, M., & Samanta, A. K. (2018). Tagatose as a Potential Nutraceutical: Production, Properties, Biological Roles, and Applications. Journal of Food Science, 83(11), 2699-2709. https://doi.org/10.1111/1750-3841.14358
Zhang, W., Yu, S., Zhang, T., Jiang, B., & Mu, W. (2016). Recent advances in D-allulose: Physiological functionalities, applications, and biological production. Trends in Food Science and Technology, 54, 127-137. https://doi.org/10.1016/j.tifs.2016.06.004
Sasahara, H., Mine, M., & Izumori, K. (1998). Production of D-talitol from D-psicose by Candida famata R28. Journal of Fermentation and Bioengineering, 85(1), 84-88. https://doi.org/10.1016/S0922-338X(97)80359-5
Takeshita, K., Ishida, Y., Takada, G., & Izumori, K. (2000). Direct Production of Allitol from D-Fructose by a Coupling Reaction Using D-Tagatose 3-Epimerase, Ribitol Dehydrogenase and Formate Dehydrogenase. Journal of Bioscience and Bioengineering, 90(5), 545-548. https://doi.org/10.1016/51389-1723(01)80038-4
Wang, L., Chen, K., Zheng, P., Huo, X., Liao, F., Zhu, L., Hu, M., & Tao, Y. (2023). Enhanced production of D-psicose from D-fructose by a redox-driven multi-enzyme cascade system. Enzyme and Microbial Technology, 163, 110172. https://doi.org/10.1016/j.enzmictec.2022.110172
Lin, B., & Tao, Y. (2017). Whole-cell biocatalysts by design. Microbial Cell Factories, 16, 106. https://doi.org/10.1186/s12934-017-0724-7
Altschul, S. F., Gish, W., Miller, W., Myers, E. W., & Lipman, D. J. (1990). Basic local alignment search tool. Journal ofMolecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Mu, W., Chu, F., Xing, Q., Yu, S., Zhou, L., & Jiang, B. (2011). Cloning, Expression, and Characterization of a D-Psicose 3-Epimerase from Clostridium cellulolyticum H10. Journal of Agricultural and Food Chemistry, 59(14), 7785-7792. https://doi.org/10.1021/jf201356q
Chan, H.-C., Zhu, Y., Hu, Y., Ko, T.-P., Huang, C.-H., Ren, F., Chen, C.-C., Ma, Y., Guo, R.-T., & Sun, Y. (2012). Crystal structures of D-psicose 3-epimerase from Clostridium cellulolyticum H10 and its complex with ketohexose sugars. Protein & Cell, 3(2), 123-131. https://doi.org/10.1007/s13238-012-2026-5

## Patentansprüche

1. Verfahren zur Herstellung einer wässerigen Lösung enthaltend _{D}-Talitol, indem aus _{D}-Fructose, welche in einer wässerigen Lösung gelöst vorliegt, durch Behandeln mit einer Epimerase in *vitro* _{D}-Psicose gebildet wird, wonach die _{D}-Psicose durch Behandeln mit einer NAD(P)H-abhängigen Oxidoreduktase *in vitro* unter Bildung von NAD(P)⁺ zu _{D}-Talitol reduziert wird und das NAD(P)⁺ mittels Formiat und einer Formiat-Dehydrogenase unter Bildung von CO₂ zu NAD(P)H reduziert wird, wonach die Epimerase, die Oxidoreduktase und die Formiat-Dehydrogenase abgetrennt werden.

2. Verfahren zur Herstellung von _{D}-Talitol, **dadurch gekennzeichnet, dass** die nach Anspruch 1 hergestellte wässerige Lösung konzentriert und das _{D}-Talitol auskristallisiert wird.

3. Verfahren nach Anspruch 1 oder 2, **dadurch gekennzeichnet, dass** die NAD(P)H-abhängige Oxidoreduktase zur Reduktion von _{D}-Psicose zu _{D}-Talitol eine Aminosäuresequenz umfasst, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

4. Verfahren zur Herstellung von _{D}-Talitol umfassend den Schritt des Behandelns von _{D}-Psicose mit einer Oxidoreduktase, die eine Aminosäuresequenz umfasst, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

5. Oxidoreduktase zur Reduktion von _{D}-Psicose zu _{D}-Talitol umfassend eine Aminosäuresequenz, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

6. Verfahren nach einem der Ansprüche 1 bis 5, **dadurch gekennzeichnet, dass** es als Eintopf-Reaktion ohne Isolierung von Zwischenprodukten durchgeführt wird.

7. Verfahren nach einem der Ansprüche 1 bis 6, **dadurch gekennzeichnet, dass** die Enzyme als Homogenat oder Lysat, Lyophilisat oder sprühgetrocknetes Enzymprodukt der entsprechenden, sie bildenden Zellen vorliegen, die Enzyme mit einem wasserlöslichen Polymer, vorzugsweise Polyethylenglycol, am N-Terminus modifiziert, oder die Enzyme in oder auf einer festen Matrix immobilisiert oder Teil eines Fusionsproteins sind.

8. Verfahren nach einem der Ansprüche 1 bis 7, **dadurch gekennzeichnet, dass** die Formiat-Dehydrogenase die Aminosäuresequenz SEQ ID Nr. 4 oder ein funktionelles Fragment davon umfasst oder daraus besteht oder durch die Nukleinsäuresequenz SEQ ID Nr. 3 kodiert wird.

9. Wässrige Lösung enthaltend _{D}-Talitol oder _{D}-Talitol erhältlich nach einem Verfahren gemäß einem der Ansprüche 1 bis 8.
